# EUROPÄISCHE PATENTSCHRIFT

(11) **EP 0 702 011 B1**
(45) Veröffentlichungstag und Bekanntmachung des Hinweises auf die Patenterteilung: **28.03.2001**
(21) Anmeldenummer: 95810518.1
(22) Anmeldetag: 16.08.1995
(51) Int. Cl.: C07D 401/12, C07D 401/14, D06P 1/62, D06M 13/358

(54) **Wasserlösliche Piperidin-Triazinverbindungen und ihre Verwendung zur Stabilisierung von Polyamidfasern**
Water soluble piperidine-triazine compounds for use as stabilizers for polyamide fibers
Composés pipéridine-triazine solubles dans l'eau et leur utilisation comme stabilisant pour des fibres de polyamide

(30) Priorität: 25.08.1994 CH 260694
(43) Veröffentlichungstag der Anmeldung: 20.03.1996
(73) Patentinhaber: Ciba Specialty Chemicals Holding Inc., 4057 Basel (CH)
(72) Erfinder: Fuso, Francesco, Dr., CH-4106 Therwil (CH); Reinert, Gerhard, Dr., CH-4123 Allschwil (CH)

(56) Entgegenhaltungen:
- EP-A- 0 094 048
- EP-A- 0 209 126
- EP-A- 0 209 127
- EP-A- 0 389 428
- EP-A- 0 459 950
- EP-A- 0 466 647
- DE-A- 2 730 449
- SEM. ORG. SYNTH. SUMMER SCH., "A.CORBELLA",18TH, 1993 Seiten 135-153, BORZATTA,V. ET AL. 'HALS as Light Stabilizer Agents Against Photodegradation of Polymers'
- PAINT INDIA, Bd. 42, Nr. 7, Juli 1992 Seiten 35-39, VALET,A. 'HALS light stabilizers: New Developments'

## Beschreibung

Die vorliegende Erfindung betrifft neue wasserlösliche Triazinverbindungen, Verfahren zu deren Herstellung und ihre Verwendung zur photochemischen und thermischen Stabilisierung von Polyamid-Fasermaterialien und -Färbungen.

Es ist bereits z.B. aus der EP-A-0 466 647 oder EP-A-0 459 950 bekannt, Polyamidfasern photochemisch und thermisch zu stabilisieren, indem man sie mit bestimmten Verbindungen aus der Klasse der sterisch gehinderten Amine, sogenannten "HALS"-Stabilisatoren, behandelt. Es hat sich jedoch gezeigt, dass die damit erreichte Stabilisierung höchsten Ansprüchen nicht immer vollauf genügt. Es besteht daher Bedarf nach Verbindungen, die einen verbesserten Schutz der Polyamid-Fasermaterialien gegen Licht und Wärmeeinwirkung bieten.

Es wurde nun gefunden, dass man mit den nachfolgend beschriebenen speziellen Triazinverbindungen eine bessere Stabilisierung von Polyamidfasermaterialien erreichen kann.

Die neuen wasserlöslichen Triazinverbindungen zeichnen sich durch eine besonders gute Affinität zur Faser und einen guten Ausziehgrad, vor allem in neutralem pH-Bereich, aus.

Gegenstand der Erfindung sind somit neue wasserlösliche Triazinverbindungen der allgemeinen Formel 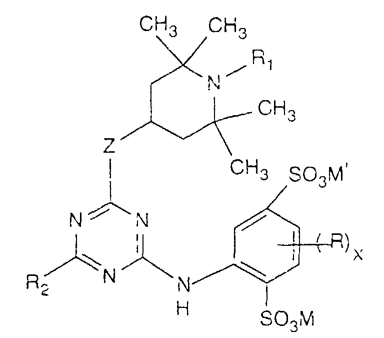 worin
R Wasserstoff; Hydroxy; Halogen: unsubstituiertes oder durch Carboxy substituiertes C₁-C₅-Alkyl; C₁-C₅-Alkoxy; C₁-C₅-Alkanoyl; Benzoyl; Mono- oder Di-C₁-C₅-alkanoylamino; Carboxy; Carbamoyl oder ein unsubstituierter oder durch C₁-C₅-Alkyl oder Halogen substituierter Phenylsulfonyl-, Phenoxy-, Phenylthio- oder Styrylrest ist;
R₁ Wasserstoff; Oxyl; Hydroxy; C₁-C₅-Alkyl; C₂-C₅-Alkenyl; C₁-C₅-Alkoxy; C₁-C₅-Alkanoyl; Benzoyl oder Benzyl ist;
R₂ durch Phenyl substituiertes Amino, wobei der Phenylrest durch unsubstituiertes oder durch Hydroxy oder Carboxy substituiertes C₁-C₅-Alkyl weiter substituiert sein kann, ist; Z -O- oder -(NR₃)- ist, wobei R₃ Wasserstoff oder C₁-C₅-Alkyl bedeutet;
M und M' voneinander unabhängig Wasserstoff; ein Alkalimetall-; Erdalkalimetall- oder Ammonium-Kation oder ein organisches Ammonium-Kation der Formel (C₁-C₄-Alkyl)ₙ(H)ₘN⁺
sind;
m 0 bis 3;
n 1 bis 4; und die Summe m + n = 4 und
x 1 oder 2 ist.

R als Halogen bedeutet z.B. Fluor, Brom und vorzugsweise Chlor.
R als C₁-C₅-Alkyl ist z.B. Methyl, Ethyl, n-Propyl, Isopropyl, n-Butyl, Isobutyl, sek.Butyl, tert.Butyl, Amyl oder Isoamyl.
R als C₁-C₅-Alkoxy ist z.B. Methoxy, Ethoxy, Isopropoxy, Isobutoxy, tert.Butoxy oder tert.Amyloxy.
R als C₁-C₅-Alkanoyl ist z.B. Formyl, Acetyl, Propionyl oder n-Butyryl.

R₁ als C₁-C₅-Alkyl ist z.B. Methyl, Ethyl, n-Propyl, Isopropyl, n-Butyl, Isobutyl, sek.Butyl, tert.Butyl, Amyl oder Isoamyl.
R₁ als C₁-C₅-Alkoxy ist z.B. Methoxy, Ethoxy, Isopropoxy, Isobutoxy, tert.Butoxy oder tert.Amyloxy.
R₁ als C₁-C₅-Alkanoyl ist z.B. Formyl. Acetyl, Propionyl oder n-Butyryl.
R₁ als C₂-C₅-Alkenyl ist z.B. Vinyl, Butenyl oder vorzugsweise Allyl.
Unter R₁ als Oxyl ist ein an den Stickstoff gebundenes Sauerstoff-radikal zu verstehen.

Als Beispiele für Alkalimetallionen M und M' seien das Lithium-. Natrium- oder Kalium-Kation genannt. Bevorzugt ist das Natrium-Kation. Beispiele für Erdalkalimetallionen sind Calcium- und Magnesium-Kation.
Für M bzw. M' als Ammonium-Kation entsprechend der Formel (C₁-C₄-Alkyl)ₙ(H)ₘN⁺ kommt Trimethylammonium oder vorzugsweise Triethylammonium in Betracht.

Von besonderem Interesse sind wasserlösliche Triazinverbindungen, bei denen in
Formel (1) Z -(NR₃)- bedeutet.

Ebenfalls von besonderem Interesse sind wasserlösliche Triazinverbindungen, bei denen in Formel (1) R Wasserstoff, Halogen, Hydroxy oder C₁-C₅-Alkyl bedeuten.

Ebenfalls von besonderem Interesse sind wasserlösliche Triazinverbindungen, bei denen in Formel (1) R₁ Wasserstoff oder C₁-C₅-Alkyl bedeuten.

Bevorzugt sind Verbindungen, bei denen in Formel (1) R₂ Phenylamino bedeutet.

Ganz besonders bevorzugt sind Verbindungen der Formel (1), worin R Wasserstoff, Halogen, Hydroxy oder C₁-C₅-Alkyl; R₁ Wasserstoff oder C₁-C₅-Alkyl, R₂ im Phenylrest unsubstituiertes oder mit C₁-C₅-Alkyl substituiertes Phenylamino und Z -(NR₃)- bedeuten.

Bevorzugt sind ferner wasserlösliche Triazinverbindungen der Formel (1), worin R Wasserstoff, Halogen, Hydroxy oder C₁-C₅-Alkyl und R₁ Wasserstoff oder C₁-C₅-Alkyl sind.

Besonders bevorzugt sind ebenfalls wasserlösliche Triazinverbindungen der Formel (1), worin R Wasserstoff, Halogen, Hydroxy, Methyl oder Ethyl, R₁ Wasserstoff und Z -(NR₃)-, worin R₃ Wasserstoff, Methyl oder Butyl bedeutet, sind.

Einen weiteren Gegenstand der Erfindung stellt das Verfahren zur Herstellung der neuen Triazinverbindungen der Formel (1) dar.

Die Herstellung der wasserlöslichen Triazinverbindungen der Formel (1) kann auf verschiedene Art und Weise erfolgen. Ausgangsverbindung ist im allgemeinen eine 2,4,6-Trihalogen-s-triazinverbindung.

Die Herstellung der erfindungsgemässen wasserlöslichen Triazinverbindungen der Formel (1) erfolgt z.B. dadurch, dass man ein Mol einer 2,4,6-Trihalogen-s-triazinverbindung nacheinander mit einem Mol der Verbindung der Formel 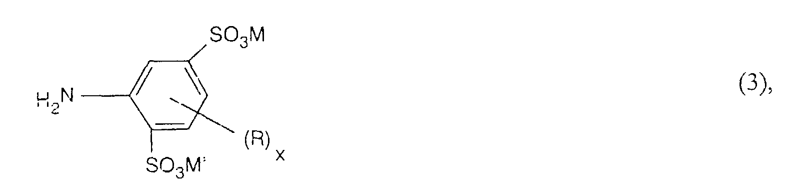 worin M, M', Z, R und x die in Formel (1) angegebene Bedeutung haben, so wie mit einem Mol der Piperidinverbindung der Formel 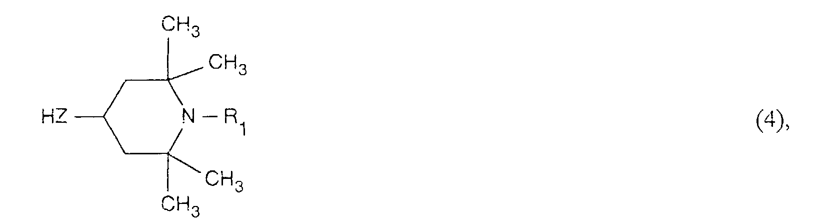 worin R₁ und Z die in Formel (1) angegebene Bedeutung haben, umsetzt, und, mit einem Mol eines Phenylamins, umsetzt, wobei die Reihenfolge der einzelnen Reaktionsschritte beliebig ist.
In der Regel geht man so vor, dass man im ersten Reaktionsschritt die 2,4,6-Trihalogen-s-triazinverbindung mit der Verbindung umsetzt, die die geringere Reaktivität aufweist.

Die Reaktionstemperatur liegt zwischen 0 und 100°C, vorzugsweise zwischen 20 und 80°C, die Reaktionszeit zwischen 1 und 20, vorzugsweise zwischen 1 und 4 Stunden.

Die bei den Kondensationsreaktionen entstehende Halogenwasserstoffsäure kann durch das Endprodukt selbst oder durch Hinzufügen einer weiteren Base, wie beispielsweise wässrigem Ammoniak, Alkalimetallhydroxiden, Alkalimetallcarbonaten,-hydrogencarbonaten oder einer organischen Base, wie beispielsweise Triethylamin, abgefangen werden. Vorzugsweise wird als Base Alkalimetallcarbonat, wie z.B. Natriumcarbonat, verwendet.

Die Umsetzungen erfolgen zweckmässigerweise in wässriger Lösung ohne den Zusatz von organischen Lösungsmitteln. Die als Ausgangsverbindungen verwendeten 2,4,6-Trihalogen-s-triazinverbindungen sind allgemein bekannt. Sie werden dabei vorzugsweise als wässrige Suspensionen eingesetzt. Eine besonders bevorzugte Ausgangsverbindung ist Cyanurchlorid.

Die übrigen für die Herstellung der Verbindungen der Formel (1) verwendeten Ausgangsverbindungen wie z.B. die Verbindungen der Formeln (3) oder (4) sind ebenfalls allgemein bekannt.

Alle Verbindungen gemäss Formel (1) werden vorzugsweise als Natriumsalze hergestellt. Dazu werden sie beispielsweise mit der äquivalenten Menge Natronlauge gelöst und als Lösung, Dispersion oder Emulsion für eine Anwendung formuliert.

Die neuen erfindungsgemässen wasserlöslichen Triazinverbindungen gemäss Formel (1) eignen sich dazu, die thermische und photochemische Stabilität von ungefärbten und gefärbten Polyamid-Fasermaterialien zu erhöhen. Die Verwendung der erfindungsgemässen Verbindungen zur Erhöhung der thermischen und photochemischen Stabilität von Polyamidfasern und -färbungen stellt somit einen weiteren Gegenstand der vorliegenden Erfindung dar.

Die neuartigen Verbindungen stellen Vertreter der Klasse der sterisch gehinderten Amine ("HALS"-Stabilisatoren) dar und können in übliche Textilveredlungsprozesse für Polyamidfasern integriert werden.
Die Verbindungen der Formel (1) werden erfindungsgemäss aus wässrigem Bad appliziert, das die Verbindungen in einer Menge, von 0,005 bis 10 Gew.-%, vorzugsweise 0,05 bis 2 Gew.-% enthält. Vorzugsweise werden die Verbindungen dem Färbebad zugefügt. Die Applikation kann dabei vor, während oder nach dem Färben, nach einem Auszieh-oder Kontinueverfahren erfolgen. Die Applikation während des Färbens ist bevorzugt.

Beim Ausziehverfahren kann das Flottenverhältnis innerhalb eines weiten Bereiches gewählt werden, z.B. 1:5 bis 1:300, vorzugsweise 1:10 bis 1:50. Man arbeitet zweckmässig bei einer Temperatur von 30 bis 130°C, vorzugsweise 50 bis 110°C.

Beim Kontinue-Verfahren beträgt der Flottenauftrag zweckmässig 30-400 Gew.-%, vorzugsweise 75-250 Gew.-%. Zur Fixierung der applizierten Farbstoffe und der bekannten und neuer. Verbindungen wird das Fasermaterial einer Hitzebehandlung unterworfen. Der Fixierprozess kann auch nach der Kaltverweilmethode erfolgen.

Die Hitzebehandlung erfolgt vorzugsweise durch ein Dämpfverfahren unter Behandlung in einem Dämpfer mit gegebenenfalls überhitztem Dampf bei einer Temperatur von 98 bis 105°C während z.B. 1 bis 7, vorzugsweise 1 bis 5 Minuten. Die Fixierung der Farbstoffe und der Verbindungen der Formel (1) gemäss dem Kaltverweilverfahren kann durch Lagerung der imprägnierten und vorzugsweise aufgerollten Ware bei Raumtemperatur (15 bis 30°C), z.B. während 3 bis 24 Stunden erfolgen, wobei die Kaltverweilzeit bekanntlich von der Art des applizierten Farbstoffes abhängig ist.

Nach Beendigung des Färbeprozesses bzw. der Fixierung werden die hergestellten Färbungen auf übliche Weise gespült und getrocknet.

Man erhält mit dem erfindungsgemässen Verfahren Polyamid-Färbungen und -fasern mit guter thermischer und photochemischer Stabilität.

Als erfindungsgemäss zu stabilisierende Färbungen kommen solche in Betracht, die durch Säure- oder Metallkomplexfarbstoffe, z.B. 1:2-Chrom-, 1:2-Kobaltkomplexfarbstoffe oder Cu-Komplexfarbstoffe aber auch Dispersions- und Reaktivfarbstoffe erzeugt werden.

Beispiele für solche Farbstoffe sind in Colour Index, 3. Auflage, 1971, Band 4, beschrieben.

Unter Polyamidfasermaterial wird synthetisches Polyamid, wie z.B. Polyamid 6, Polyamid 6,6 oder Polyamid 12, sowie modifiziertes Polyamid, z.B. basisch anfärbbares Polyamid verstanden. Neben den reinen Polyamidfasern kommen vor allem auch Fasermischungen aus Polyurethan und Polyamid in Betracht, so z.B. Trikotmaterial aus Polyamid/Elastan-faser im Mischungsverhältnis 70:30. Grundsätzlich kann das reine oder gemischte Polyamidfasermaterial in den verschiedensten Verarbeitungsformen vorliegen, wie z.B. als Faser, Garn, Gewebe, Gewirke, Vlies oder Flormaterial.

Das vorliegende Verfahren eignet sich besonders vorteilhaft zur Behandlung von Polyamidfasermaterial, das Licht und Hitze ausgesetzt wird und z.B. als Autopolsterstoff oder Teppich Verwendung findet.

Die folgenden Beispiele veranschaulichen die Erfindung. Teile bedeuten Gewichtsteile und Prozente Gewichtsprozente.

### Beispiel 1:

a) In eine pH-neutrale Lösung von 0,05 Mol 2-(2,4-Dichlor-s-triazin-6-ylamino)benzol-1,4-disulfonsäure in 150 ml Wasser werden 4,65 g Anilin eingetragen. Man erwärmt die Reaktionsmischung unter Rühren auf 35° C und hält den pH-Wert durch gleichzeitiges Zutropfen von 2M Natronlauge bei etwa 7. Man rührt solange, bis die Kondensation beendet ist, und kein Anilin mehr nachgewiesen werden kann.
b) Zu der oben beschriebenen Lösung trägt man 10,61 g 4-Butylamino-2,2,6,6-tetramethylpiperidin ein, erhitzt die Reaktionsmischung auf 85° C und hält sie bei dieser Temperatur während 20 Stunden. Anschliessend wird der Kolbeninhalt auf 40° C abgekühlt, mit 2M Salzsäure bis zu einem pH-Wert von 5 angesäuert und mit 40 g NaCl ausgesalzt. Der Niederschlag wird abfiltriert, mit 60 ml einer 15%-igen Natriumchlorid-Lösung portionensweise gewaschen und im Vacuum bei 70° C getrocknet. Man erhält ein Pulver der Formel

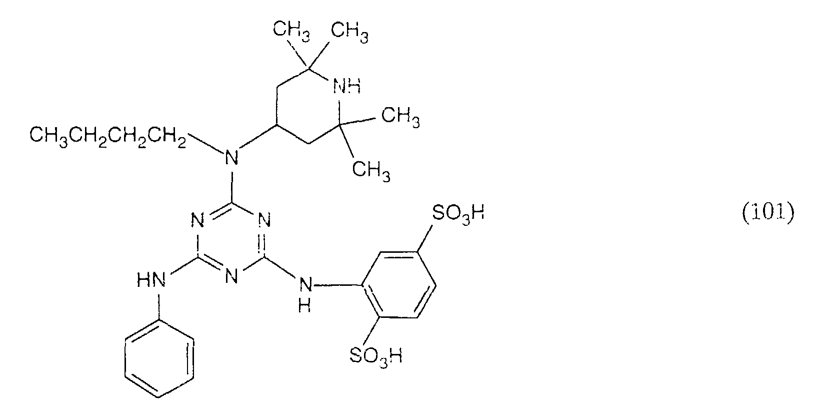

### Beispiel 2:

Verfährt man wie in Beispiel 1 beschrieben, setzt jedoch im Schitt b) anstelle von 10,61 g 4-Butylamino-2,2,6,6-tetramethylpiperidin 7,88 g 4-Amino-2,2,6,6-tetramethylpiperidin ein, so erhält man eine Verbindung der Formel

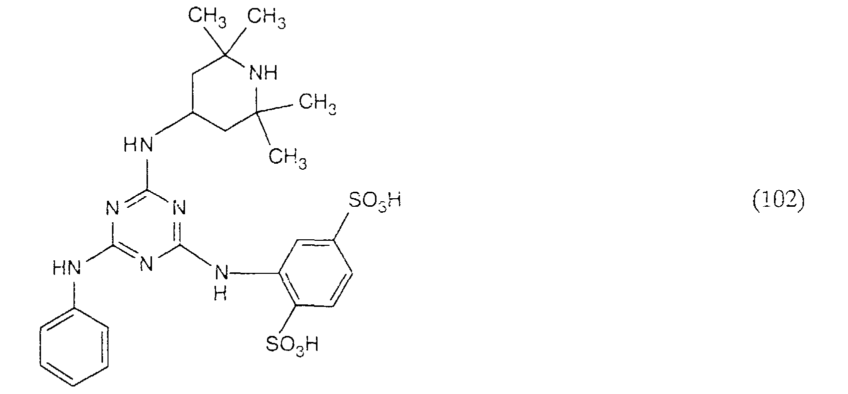

### Beispiel 3:

a) Zu einer Lösung von 0,1 Mol 2-(2,4-Dichlor-s-triazin-6-ylamino)benzol-1,4-disulfonsäure in 300 ml Wasser wird innerhalb von 20 Minuten eine Lösung von 10,7 g p-Toluidin in 50 ml Dioxan zugetropft. Durch gleichzeitiges Zutropfen von 15%-iger Sodalösung hält man einen pH-Wert von ungefähr 7 ein. Zur Vervollständigung der Reaktion rührt man die Reaktionsmischung während einer Stunde bei 35° C nach. Anschliessend kühlt man den Kolbeninhalt auf Raumtemperatur ab und teilt die Reaktionslösung in 3 gleiche Teile auf.
b) Zu einem Teil der oben beschriebenen Lösung werden rasch 5,2 g
   4-Amino-2,2,6,6-tetramethylpiperidin eingetragen und die Reaktionstemperatur auf 80-85° C erhöht. Man rührt die Reaktionsmischung während 16 Stunden aus, kühlt auf 10° C ab und säuert mit 2M Salzsäure bis zu einem pH-Wert von 3 an. Anschliessend wird die Reaktionsmischung mit 50 ml einer 10%-igen Natriumchlorid-Lösung versetzt. Der entstandene Niederschlag wird abfiltriert, mit wenig Wasser gewaschen und im Vacuum bei 70° C getrocknet. Man erhält ein Pulver der Formel

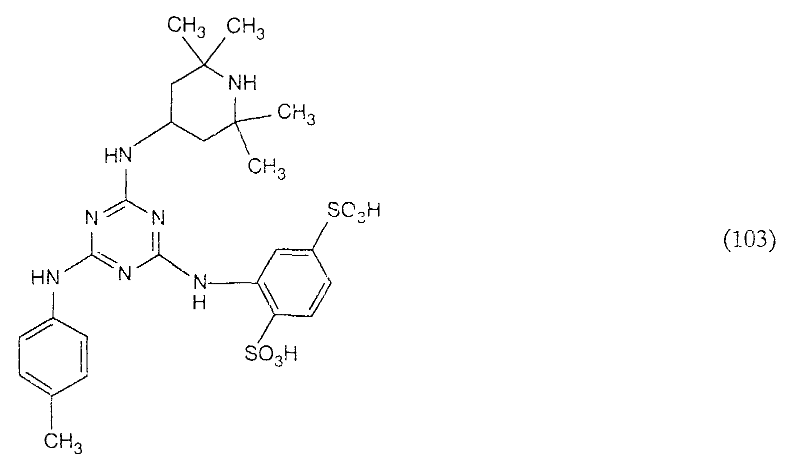

### Beispiel 4:

Verfährt man wie in Beispiel 3b beschrieben, setzt jedoch anstelle von 5,2 g 4-Amino-2,2,6,6-tetramethylpiperidin 5,6 g 4-Methylamino-2,2,6,6-tetramethylpiperidin ein, so erhält man eine Verbindung der Formel

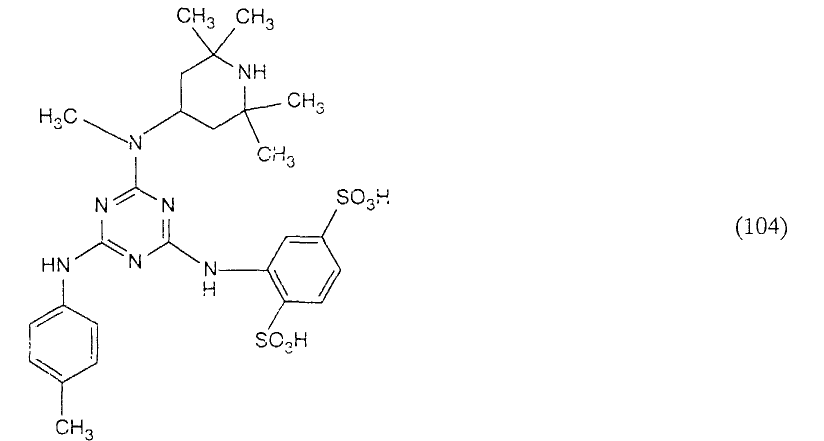

### Beispiel 5:

Verfährt man wie in Beispiel 3b beschrieben, setzt jedoch anstelle von 5,2 g
4-Amino-2,2,6,6-tetramethylpiperidin 7,0 g 4-Butylamino-2,2,6,6-tetramethylpiperidin ein, so erhält man eine Verbindung der Formel

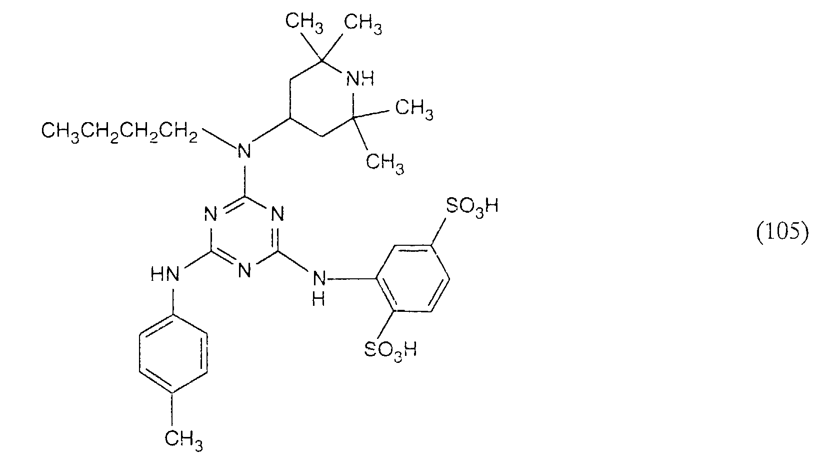

Auf analoge Weise wie in den Beispielen 1 bis 5 beschrieben können auch folgende Verbindungen hergestellt werden:

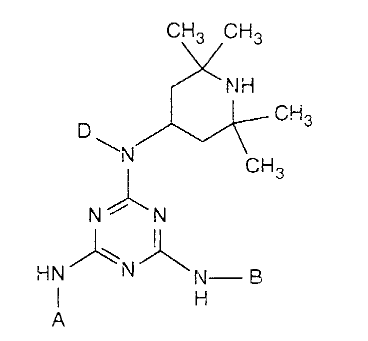

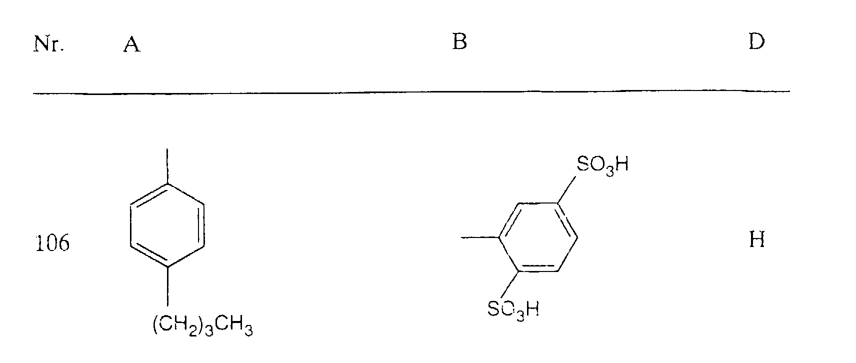

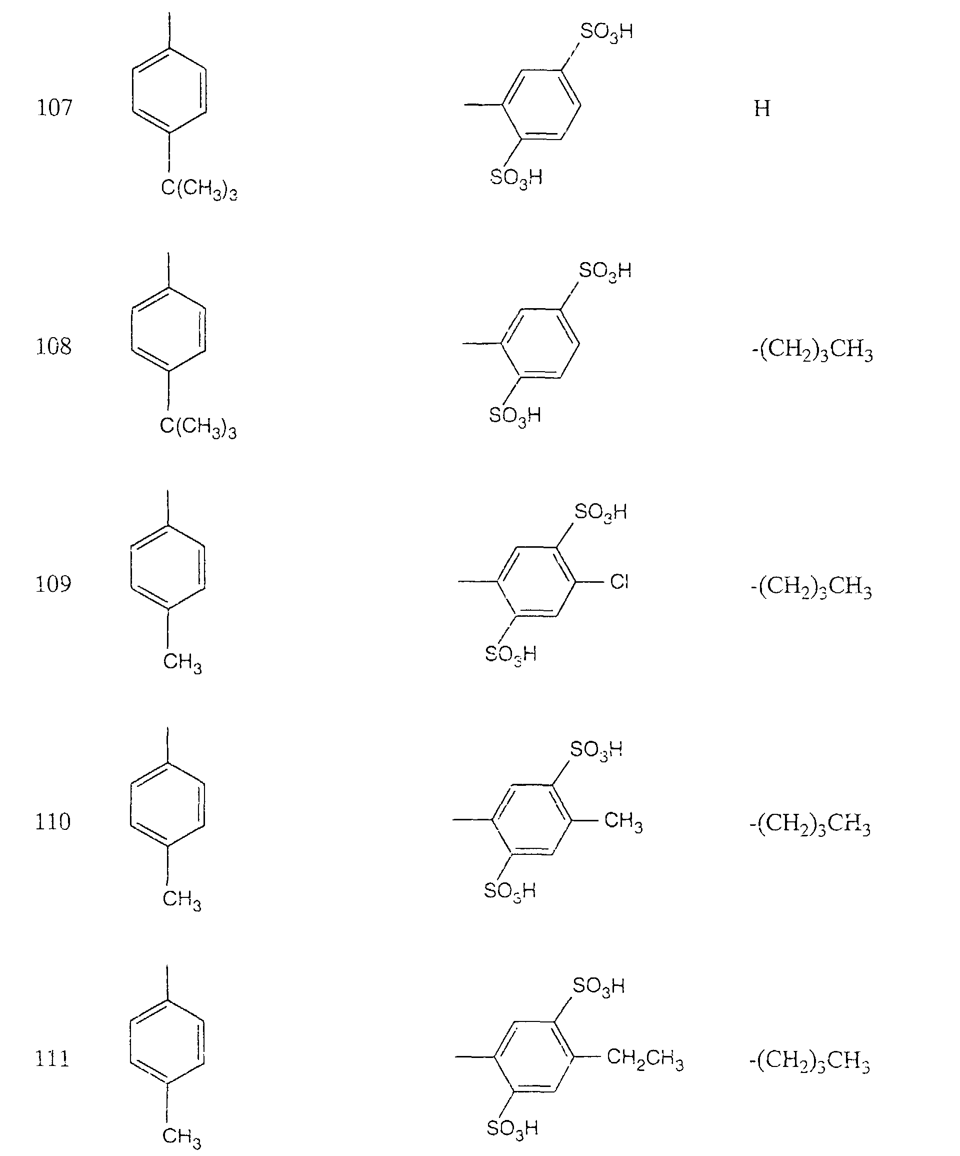

### Beispiel 6:

In einem ®AHIBA-Färbeapparat wird bei einem Flottenverhältnis von 1:30 ein Muster von 10 g einer Polyamid 6-Maschenware gefärbt. Die Färbeflotte enthält folgende Zusätze: 0,5g/l Mono-Na-phosphat, 1,5 g/l Di-Na-phosphat, 0,04 Gew.-% Farbstoff der Formel (I), 0,002 Gew.-% Farbstoff der Formel (II) und 0,001 %, jeweils bezogen auf das zu färbende Material, Farbstoff der Formel (III) in gelöster Form: 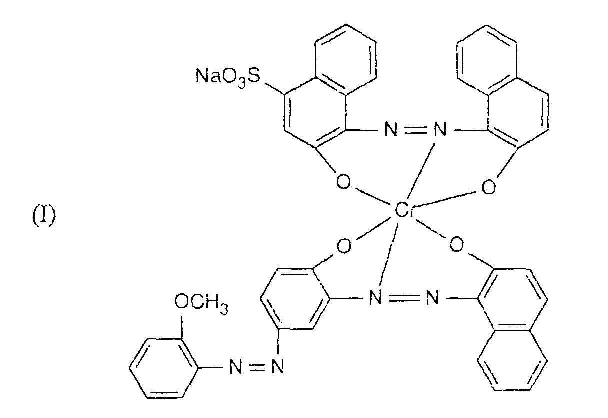 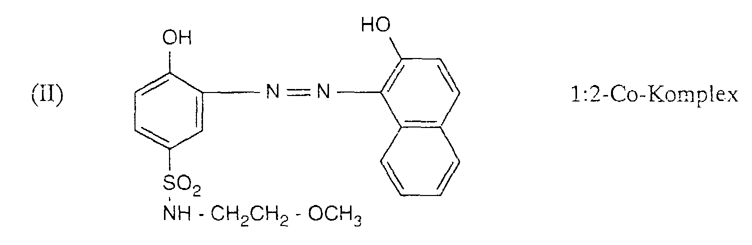 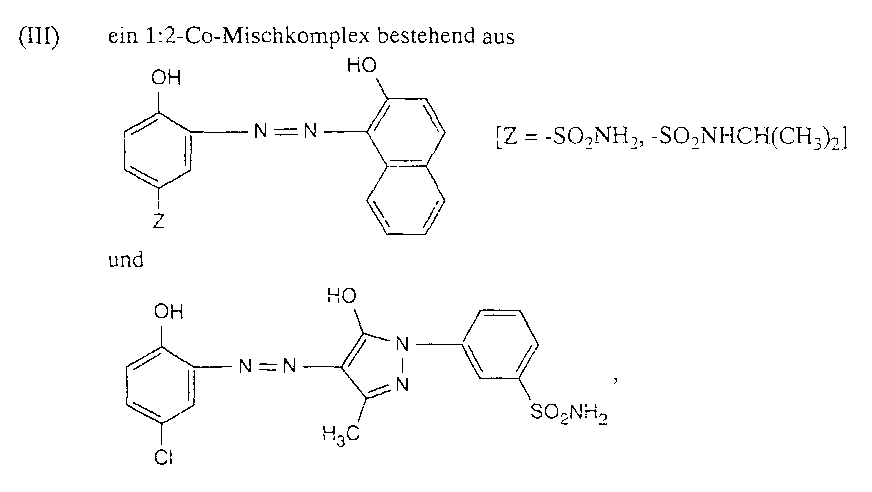 sowie 0,25 Gew.-%, bezogen auf das Färbegut, der Verbindung der Formel (102)

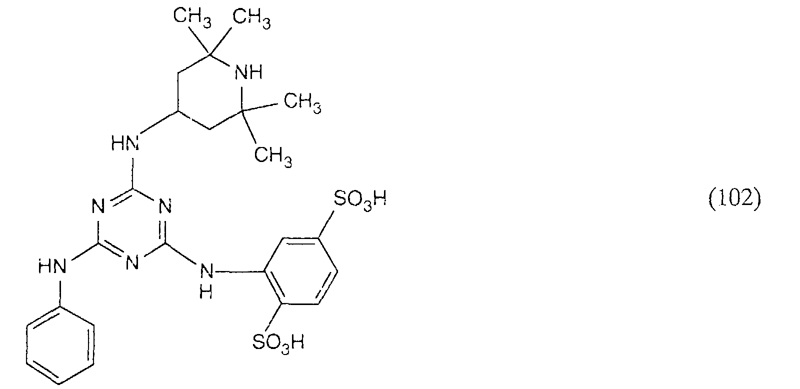

Die Flotte wird auf 45°C erwärmt. Nach Zugabe des Textilmaterials behandelt man 10 Minuten bei dieser Temperatur und erwärmt mit 2°C/Minute auf 95°C. Nach einer Färbezeit von 15 Minuten bei 95°C werden 2 Gew.-%, bezogen auf das Färbegut, 80 %ige Essigsäure zugesetzt und weitere 30 Minuten gefärbt. Danach kühlt man auf 60°C ab, spült das gefärbte Material mit heissem Wasser und trocknet es.
Man erhält Färbungen mit sehr guten Lichtechtheiten.

## Patentansprüche

1. Wasserlösliche Triazinverbindungen der Formel 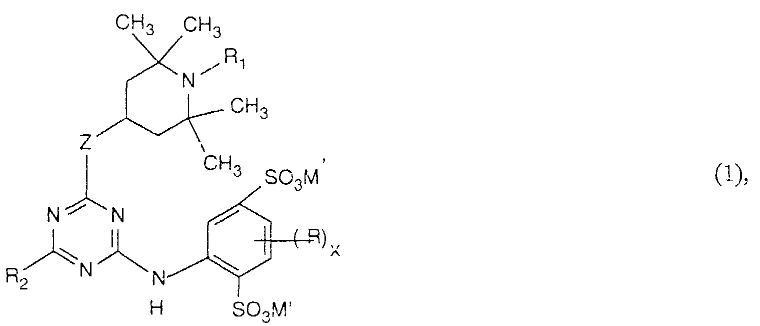 worin
R Wasserstoff; Hydroxy; Halogen; unsubstituiertes oder durch Carboxy substituiertes C₁-C₅-Alkyl; C₁-C₅-Alkoxy; C₁-C₅-Alkanoyl; Benzoyl; Mono- oder Di-C₁-C₅-alkanoylamino; Carboxy; Carbamoyl oder ein unsubstituierter oder durch C₁-C₅-Alkyl oder Halogen substituierter Phenylsulfonyl-, Phenoxy-, Phenylthio- oder Styrylrest ist;
R₁ Wasserstoff; Oxyl; Hydroxy; C₁-C₅-Alkyl; C₂-C₅-Alkenyl; C₁-C₅-Alkoxy; C₁-C₅-Alkanoyl; Benzoyl oder Benzyl ist;
R₂ durch Phenyl substituiertes Amino, wobei der Phenylrest durch Hydroxy oder Carboxy substituiertes C₁-C₅-Alkyl weiter substituiert sein kann, ist;
Z -O- oder -(NR₃)- ist, wobei R₃ Wasserstoff oder C₁-C₅-Alkyl bedeutet;
M und M' voneinander unabhängig Wasserstoff; ein Alkalimetall-; Erdalkalimetall- oder Ammonium-Kation oder ein organisches Ammonium-Kation der Formel
(C₁-C₄-Alkyl)ₙ(H)ₘN+
sind;
m 0 bis 3;
n 1 bis 4; und die Summe m + n = 4 und
x 1 oder 2 ist.

2. Wasserlösliche Triazinverbindungen gemäss Anspruch 1, dadurch gekennzeichnet, dass R Wasserstoff, Halogen, Hydroxy oder C₁-C₅-Alkyl ist.

3. Wasserlösliche Triazinverbindungen gemäss einem der Ansprüche 1 oder 2, dadurch gekennzeichnet, dass R₁ Wasserstoff oder C₁-C₅-Alkyl ist.

4. Wasserlösliche Triazinverbindungen gemäss einem der Ansprüche 1 bis 5, dadurch gekennzeichnet, dass Z -(NR₃)- bedeutet, wobei R₃ Wasserstoff oder C₁-C₅-Alkyl ist.

5. Wasserlösliche Triazinverbindungen gemäss Anspruch 1, dadurch gekennzeichnet, dass R Wasserstoff, Halogen, Hydroxy oder C₁-C₅-Alkyl, R₁ Wasserstoff oder C₁-C₅-Alkyl sind.

6. Wasserlösliche Triazinverbindungen gemäss Anspruch 1, dadurch gekennzeichnet, dass R Wasserstoff, Halogen, Hydroxy, Methyl oder Ethyl, R₁ Wasserstoff und Z -(NR₃)-, worin R₃ Wasserstoff, Methyl oder Butyl bedeutet, sind.

7. Verfahren zur Herstellung der Triazinverbindungen der Formel 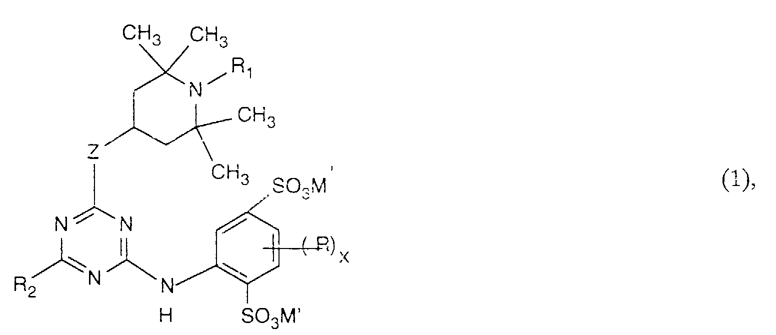 worin
R, R₁, R₂, Z, M, M', und x die in Anspruch 1 angegebenen Bedeutungen haben, dadurch gekennzeichnet, dass man ein Mol einer 2,4,6-Trihalogen-s-triazinverbindung nacheinander mit einem Mol der Verbindung der Formel 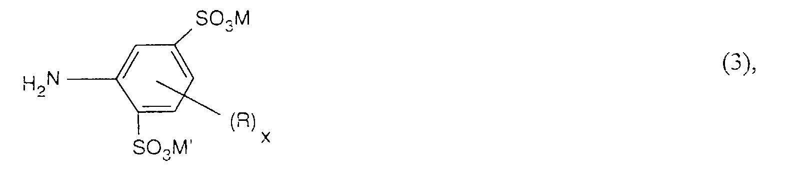 worin M, M', Z, R und x die in Formel (1) angegebene Bedeutung haben, so wie mit einem Mol der Piperidinverbindung der Formel 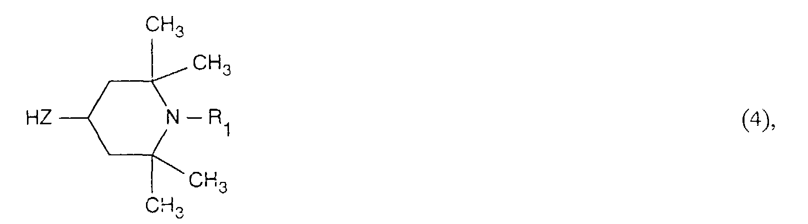 worin R₁ und Z die in Formel (1) angegebene Bedeutung haben, umsetzt, und mit einem Mol eines Phenylamins umsetzt, wobei die Reihenfolge der einzelnen Reaktionsschritte beliebig ist.

8. Verfahren zur photochemischen und thermischen Stabilisierung von Polyamidfasermaterialien, dadurch gekennzeichnet, dass man gefärbte oder ungefärbte Polyamid-Fasermaterialien mit wasserlöslichen Triazinverbindungen der Formel (1) gemäss Anspruch 1 behandelt.

9. Verwendung der Triazinverbindungen gemäss einem der Ansprüche 1 bis 6 zur photochemischen und thermischen Stabilisierung von Polyamid-Fasermaterialien und -Färbungen.

10. Das mit den Triazinverbindungen gemäss einem der Ansprüche 1 bis 6 behandelte Fasermaterial.

## Claims

1. A water-soluble triazine compound of formula 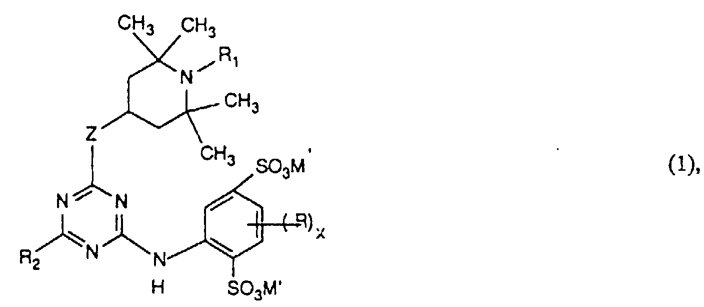 wherein
R is hydrogen, hydroxy; halogen; unsubstituted or carboxy-substituted C₁-C₅alkyl; C₁-C₅alkoxy; C₁-C₅alkanoyl; benzoyl; mono- or di-C₁-C₅alkanoylamino; carboxy; carbamoyl or an unsubstituted or a C₁-C₅alkyl- or halogen-substituted phenylsulfonyl, phenoxy, phenylthio or styryl radical;
R₁ is hydrogen; oxyl; hydroxy; C₁-C₅alkyl; C₂-C₅alkenyl; C₁-C₅alkoxy; C₁-C₅alkanoyl; benzoyl or benzyl;
R₂ is phenyl-substituted amino in which the phenyl moiety may be further substituted by hydroxy- or carboxy-substituted C₁-C₅alkyl;
Z is -O- or -(NR₃)-, where R₃ is hydrogen or C₁-C₅alkyl;
M and M' are each independently of the other hydrogen, an alkali metal cation, an alkaline earth metal cation or an ammonium cation or an organic ammonium cation of formula (C₁-₄alkyl)ₙ(H)ₘN⁺;
m is 0 to 3;
n is 1 to 4; and the sum of m + n = 4, and
x is 1 or 2.

2. A water-soluble triazine compound according to claim 1, wherein R is hydrogen, halogen, hydroxy or C₁-C₅alkyl.

3. A water-soluble triazine compound according to either claim 1 or claim 2, wherein R₁ is hydrogen or C₁-C₅alkyl.

4. A water-soluble triazine compound according to any one of claims 1 to 3, wherein Z is -(NR₃)-, where R₃ is hydrogen or C₁-C₅alkyl.

5. A water-soluble triazine compound according to claim 1, wherein R is hydrogen, halogen, hydroxy or C₁-C₅alkyl, R₁ is hydrogen or C₁-C₅alkyl.

6. A water-soluble triazine compound according to claim 1, wherein R is hydrogen, halogen, hydroxy, methyl or ethyl, R₁ is hydrogen, and Z is -(NR₃)-, in which R₃ is hydrogen, methyl or butyl.

7. A process for the preparation of a triazine compound of formula 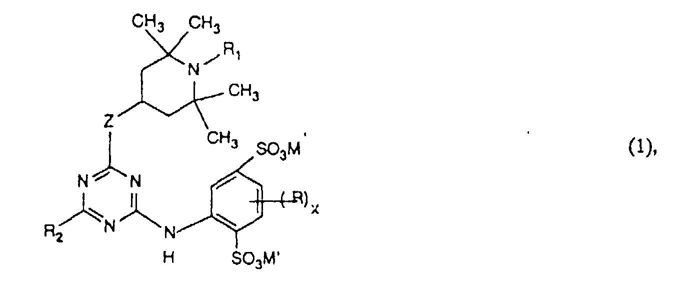 wherein
R, R₁, R₂, Z, M, M', and x are as defined in claim 1, which comprises reacting 1 mol of a 2,4,6-trihalo-s-triazine compound, in succession, with 1 mol of the compound of formula 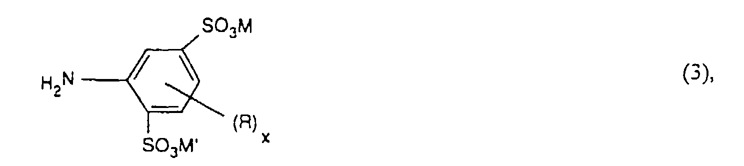 wherein M, M', Z, R and x are as defined with respect to formula (1), and with 1 mol of the piperdine compound of formula 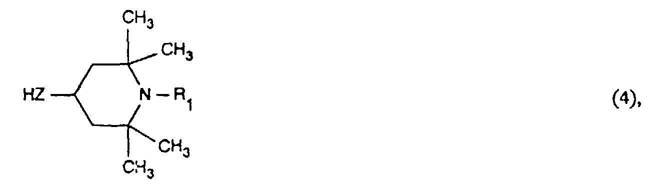 wherein R₁ and Z are as defined with respect to formula (1), and with 1 mol of a phenylamine, the sequence of the individual reaction steps being arbitrary.

8. A process for the photochemical and thermal stabilisation of polyamide fibre materials, which comprises treating dyed or undyed polyamide fibre materials with a water-soluble triazine compound of formula (1) according to claim 1.

9. Use of a triazine compound according to any one of claims 1 to 6 for the photochemical and thermal stabilisation of polyamide fibre materials and their dyeings.

10. Fibre material treated with a triazine compound according to any one of claims 1 to 6.

## Revendications

1. Composés hydrosolubles de la triazine de formule 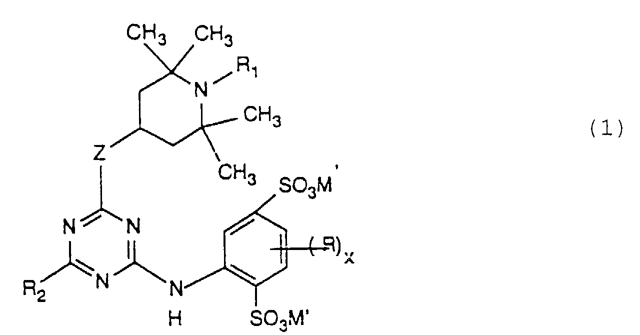 où
R représente un atome d'hydrogène ; des groupes hydroxy ; halogène ; benzoyle ; mono- ou di-(alcanoyl en C₁-C₅)amino ; alkoxy en C₁-C₅ ; alcanoyle en C₁-C₅ ; alkyle en C₁-C₅ non substitué ou substitué par un substituant carboxy ; carboxy ; carbamoyle ou un reste phénoxy, phénylthio, styryle ou phénylsulfonyle non substitué ou substitué par des substituants alkyle en C₁-C₅ ou halogène ;
R₁ représente un atome d'hydrogène ; des groupes oxyle ; hydroxy ; alkyle en C₁-C₅ ; alcényle en C₂-C₅ ; alkoxy en C₁-C₅ ; alcanoyle en C₁-C₅ ; benzoyle ou benzyle ;
R₂ représente un groupe amino substitué par un reste phényle, le reste phényle pouvant être non substitué ou substitué encore par des substituants hydroxy ou carboxy ;
Z représente -O- ou -(NR₃)-, R₃ représentant un atome d'hydrogène ou un groupe alkyle en C₁-C₅ ; M et M' représentent, indépendamment l'un de l'autre, un atome d'hydrogène, un cation métal alcalin ; alcalino-terreux ou ammonium ou un cation ammonium organique de formule (alkyl en C₁-C₄)ₙ(H)ₘN⁺ ;
m va de 0 à 3 ;
n va de 1 à 4 ; et la somme m + n = 4 et
x vaut 1 ou 2.

2. Composés hydrosolubles de la triazine selon la revendication 1, caractérisés en ce que R représente un atome d'hydrogène, un atome d'halogène ou un groupe alkyle en C₁-C₅.

3. Composés hydrosolubles de la triazine selon l'une des revendications 1 ou 2, caractérisés en ce que R₁ représente un atome d'hydrogène ou un groupe alkyle en C₁-C₅.

4. Composés hydrosolubles de la triazine selon l'une des revendications 1 à 5, caractérisés en ce que Z représente un groupe -(NR₃)-, R₃ représentant un atome d'hydrogène ou un groupe alkyle en C₁-C₅.

5. Composés hydrosolubles de la triazine selon la revendication 1, caractérisés en ce que R représente un atome d'hydrogène, un atome d'halogène, des groupes hydroxy ou alkyle en C₁-C₅, R₁ représente un atome d'hydrogène ou un groupe alkyle en C₁-C₅.

6. Composés hydrosolubles de la triazine selon la revendication 1, caractérisés en ce que R représente un atome d'hydrogène, un atome d'halogène, des groupes hydroxy, hydroxy, méthyle ou éthyle, R₁ eprésente un atome d'hydrogène et Z représente un groupe -(NR₃)-, où R₃ représente un atome d'hydrogène, des groupes méthyle ou butyle.

7. Procédé pour la préparation des composés de la triazine de formule 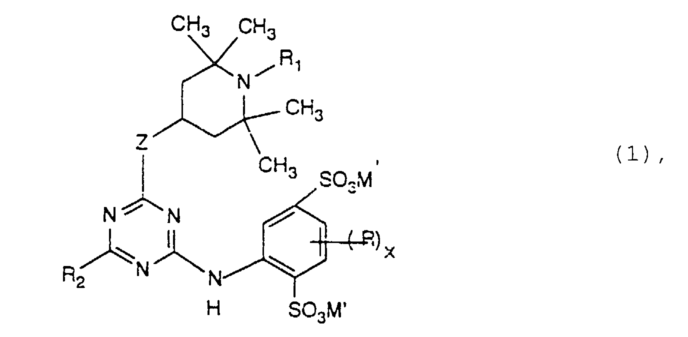 où
R, R₁, R₂, Z, M, M' et x possèdent la signification donnée à la revendication 1, caractérisé en ce qu'on fait réagir une mole d'un composé de la 2.4.6-trihalogéno-s-triazine successivement sur une mole du composé de formule 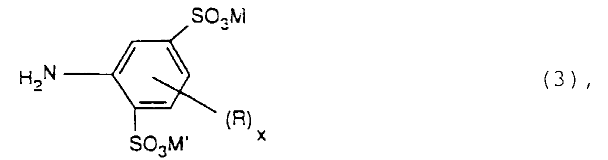 où M, M', Z, R et x possèdent la signification donnée à la formule (1), ainsi que sur une mole du composé de la pipéridine de formule 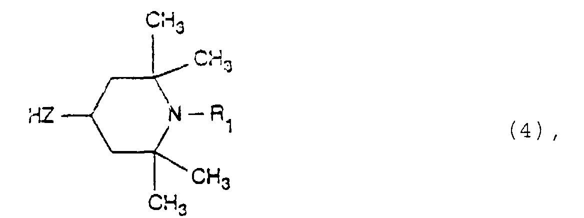 où R₁ et Z possèdent la signification donnée à la formule (1), et on fait réagir sur une mole d'une phénylamine, la succession des différents stades réactionnels étant arbitraire.

8. Procédé pour la stabilisation photochimique et thermique des matières fibreuses polyamides, caractérisé en ce qu'on traite des matières fibreuses polyamides teintes et non teintes par des composés hydrosolubles de la triazine de formule (1) selon la revendication 1.

9. Utilisation des composés de la triazine selon l'une des revendications 1 à 6 pour la stabilisation photochimique et thermique des matières fibreuses polyamides et des teintures de matières fibreuses polyamides.

10. La matière fibreuse traitée par l'une des revendications 1 à 6.
